# EUROPÄISCHE PATENTSCHRIFT

(11) **EP 3 259 007 B1**
(45) Veröffentlichungstag und Bekanntmachung des Hinweises auf die Patenterteilung: **23.01.2019**
(21) Anmeldenummer: 16703044.4
(22) Anmeldetag: 08.01.2016
(51) Int. Cl.: A61M 16/10, A62B 7/10, A63B 23/02, B01D 46/00, F24F 3/16, A62B 19/00, A61M 16/04

(54) **FILTER SOWIE VORRICHTUNG ZUR BEATMUNG MIT FILTER**
FILTER AND DEVICE FOR ARTIFICIAL RESPIRATION HAVING A FILTER
FILTRE ET DISPOSITIF RESPIRATOIRE À FILTRE

(30) Priorität: 16.02.2015 DE 102015002052
(43) Veröffentlichungstag der Anmeldung: 27.12.2017
(73) Patentinhaber: Weinmann Emergency Medical Technology GmbH + Co. KG, 22525 Hamburg (DE)
(72) Erfinder: SOMMER, Andreas, 22417 Hamburg (DE); PULLA, Matthias, 22459 Hamburg (DE); HEIN, Stefan, 22529 Hamburg (DE)
(74) Vertreter: Klickow & Wetzel PartGmbB
(86) Internationale Anmeldenummer: PCT/DE2016/000009
(87) Internationale Veröffentlichungsnummer: WO 2016/131434

(56) Entgegenhaltungen:
- EP-A1- 1 537 903
- EP-A1- 2 572 747
- EP-A2- 0 462 412
- WO-A1-2009/149507
- DE-A1-102010 047 565
- US-A1- 2003 070 406
- US-B1- 6 780 217

## Beschreibung

Die wird in den angehängten Ansprüchen definiert und betrifft einen Hygienefilter für eine Vorrichtung zur Beatmung, aufweisend wenigstens einen Filter zur Filterung von Mikroorganismen und/oder Feststoffpartikeln aus einem Atemluftvolumenstrom, bei dem das Filtermaterial als Abdichtungsmittel gegenüber dem atemluftvolumenstromleitenden Element ausgebildet ist, sodass das Risiko auftretender Nebenluft reduziert ist.
Weiterhin betrifft die Erfindung eine Vorrichtung zur Beatmung aufweisend eine Halterungsvorrichtung innerhalb des Luftansaugbereiches.
Filtereinrichtungen werden typischerweise bei pneumatischen Einrichtungen verwendet, um ein Ansaugen von Partikeln zu verhindern. Häufig sind in Strömungsrichtung hinter der Filtereinrichtung Gebläse, Ventilatoren oder Kompressoren angeordnet. In Strömungsrichtung ist typischerweise ein Feinfilter oder Grobfilter angeordnet, um gröbere Verunreinigungen zurückzuhalten.

Ein bakteriendichter Filter wird im Bereich einer Schlauchleitung zwischen einem Beatmungsgerät und einer Atemmaske eines Patienten eingesetzt, um aus einer Umgebung angesaugte Verunreinigungen zurückzuhalten. Zudem dient ein solcher Filter in der Schlauchleitung dazu, eine Rückatmung von möglicherweise bakteriell kontaminierter Ausatemluft in das Beatmungsgerät zu verhindern. Nachteilig an bekannten Lösungen ist, dass Bakterien und Viren aus der Umgebungsluft durch den Ansaugbereich in das Beatmungsgerät gelangen können. Ein derart kontaminiertes Gerät muss aufwändig hygienisch aufbereitet werden. Sie schützen zumindest den Patienten vor einer Kontamination, nicht jedoch das Beatmungsgerät.

Bei der Anwendung im Zusammenhang mit medizintechnischen Geräten können jedoch gesundheitliche Beeinträchtigungen von Patienten auftreten, die die betreffenden Geräte benutzen, wenn Keime oder Viren aus der Umgebungsluft oder aus der Vorrichtung zur Beatmung durch die Beatmung in die Atemwege des Patienten gelangen. Atemwegsinfekte (Tuberkulose, Tracheobronchitis oder Pneumonie) stellen die größte Gruppe der im Krankenhaus, Intensivbereich und Rettungsdienst auftretenden Infektionen dar. Länderübergreifende Ausbreitung von Infektionskrankheiten, die sog. Pandemien, werden zunehmend bedeutender und gewinnen immer mehr Beachtung, da sich diese über unsere heutigen schnellen Transportwege u.a. durch den Flugtourismus sehr schnell ausbreiten können. Deutlich wurde dieser Effekt auch an der Verbreitung von SARS im Jahre 2003. Als jüngstes Beispiel kann auch die Influenza-Infektion der sog. Schweinegrippe genannt werden.

Die Übertragung von Keimen kann über kontaminierte Beatmungsgeräte erfolgen. Die Geräte werden dabei beispielsweise durch Rückatmung des Patienten in das Gerät mit Keimen kontaminiert oder auch, im sogenannten Air-Mix-Modus (bei dem Umgebungsluft eingezogen wird), durch Keime die sich in der Umgebungsluft befinden und vom Gerät angesaugt werden. Kontaminierte Geräte müssen je nach Bautyp aufwändig hygienisch aufbereitet oder ausgetauscht werden.

Um eine Keimübertragung zwischen Patienten zu vermeiden und eine teure hygienische Aufbereitung des Gerätes einzusparen, wird ein auswechselbarer, bakterien- und virendichter Filter für den Luftansaugbereich einer Vorrichtung zur Beatmung eingesetzt, welches zudem eine Filterwechselanzeige aufweist, die dem Anwender die Wechselintervalle vorgibt. Geräte mit Filtersystem für Viren und Bakterien helfen Kosten im Gesundheitswesen einzusparen und - durch den Schutz vor Kontamination - die Patientensicherheit zu erhöhen.

Das Ziel, die Keimübertragung zwischen Patienten zu vermeiden, ohne die eingesetzten Geräte hygienisch aufzubereiten, kann durch die Verwendung auswechselbarer, bakterien- und virendichter Filter für den Luftansaugbereich einer Vorrichtung zur Beatmung erreicht werden, wenn sichergestellt ist, dass zum einen die unerwünschten Bakterien und Viren zuverlässig von dem eingesetzten Filtermaterial zurückgehalten werden und sich zum anderen den Mikroorganismen kein anderer Weg eröffnet den Filter zu passieren.

Aus der DE 10 2010 047 565 A1 ist bereits ein Hygienefilter bekannt, der zur Verwendung im Bereich einer Vorrichtung zur Beatmung vorgesehen ist. Der Hygienefilter ist mit einem Filter zur Filterung von Mikroorganismen oder Feststoffpartikeln ausgestattet. Zur Verringerung des Risikos eines Auftretens von Nebenluft wird ein Abdichtungsmittel verwendet.

Vergleichbare Konstruktion werden auch in der EP 1537903 A1, der US 2003/070 406 A1, der EP 2572747 A1 sowie der EP 0462412 A2 beschrieben. Weitere Hygienefilter sind aus der WO 2009/149 507 A1 sowie der US 6 780 217 B1 bekannt.

Aufgabe der vorliegenden Erfindung ist es, ein für Mikroorganismen wie Bakterien und Viren sowie Feststoffpartikel verschiedener Größe geeignetes Filtermaterial und dessen konstruktive Gestaltung bereitzustellen, das in einer Vorrichtung zur Beatmung der einleitend genannten Art verwendbar und derart gestaltet ist, dass Mikroorganismen und/oder Feststoffpartikel im Wesentlichen aus dem durch den Filter durchgeleiteten Volumenstrom abgeschieden werden.

Diese Aufgabe wird dadurch gelöst, dass ein bakterien- viren- und, oder partikeldichtes Filtermaterial in Kombination mit einer auf das Filtermaterial abgestimmten Filterhalterung derart und austauschbar ausgestaltet ist, dass Organismen und/oder Partikel die Filterungsstelle innerhalb des Luftansaugbereiches einer Vorrichtung zur Beatmung im Wesentlichen nicht passieren können, sodass ein verunreinigungsarmer Raum in Strömungsrichtung nach der Filterungsstelle unterstützt wird.
Die erfindungsgemäße Lehre erkennt, dass die Situation eines verunreinigungsarmen Raumes in Strömungsrichtung nach der Filterungsstelle insbesondere dann unterstützt ist, wenn das Filtermaterial die erforderlichen Filterungseigenschaften aufweist und, oder keine Volumenstromteile an dem Filtermaterial vorbeiströmen und somit ungefiltert bleiben und, oder der Filter oder das Filtermaterial nach Erreichen eines für die Filterungseigenschaften ungünstigen Verschmutzungsgrades austauschbar angeordnet ist.

In einer Ausführungsvariante ist eine Filterhalterung für ein bakterien- und virendichtes Filtermaterial in einem Gerätegehäuse im Luftansaugbereich gehaltert und relativ zu diesem austauschbar angeordnet.
Durch eine mindestens teilweise asymmetrische Ausbildung der Filterhalterung und die wenigstens temporäre Verbindung von Filter und Filterrahmen zu einem Hygienefiltersystem ist es nicht möglich, das Hygienefiltersystem in falscher Positionierung im Bereich des Gerätegehäuses zu fixieren.
Eine kippsichere Haltung wird dadurch ermöglicht, dass das Gerätegehäuse im Bereich der Aufnahme für das Hygienefiltersystem eine Auflagefläche für das Hygienefiltersystem bereitstellt.
Zu einer sicheren Fixierung des Hygienefiltersystems in einem vorgesehenen Betriebszustand trägt es bei, dass im Wesentlichen gegenüberliegend zur Abstützung ein Rastelement zur Fixierung des Hygienefiltersystems im Bereich des Gerätegehäuses angeordnet ist.

Eine einfache manuelle Handhabbarkeit wird dadurch unterstützt, dass das Rastelement mindestens bereichsweise federnd verformbar ausgebildet ist.
Eine gute Zugänglichkeit für einen Filterwechsel wird dadurch erreicht, dass die Filterhalterung mindestens bereichsweise als eine Schwenkhalterung ausbildet.

Je nach verwendetem Filtermaterial ist es möglich, dass das Hygienefiltersystem ohne Rahmen, Filtergehäuse oder dergleichen nur aus dem Filtermaterial ausgebildet ist, dass in die Filterhalterung in einer Vorrichtung zur Beatmung der einleitend genannten Art fixierbar ist. In diesem Ausführungsbeispiel muss das Filtermaterial neben der geforderten Filtereigenschaft und mechanisch-strukturellen Festigkeit auch geeignet sein, gegenüber der Filterhalterung zuverlässig abzudichten, um Nebenluft zu vermeiden.
Eine bevorzugte Anwendung besteht darin, dass das Gerätegehäuse als Teil eines Notfall-Beatmungsgerätes ausgebildet ist.
Eine hohe Funktionalität bei gleichzeitig hoher mechanischer Stabilität wird in einer der möglichen Ausführungsvarianten dadurch erreicht, dass die Filterhalterung klappenartig ausgebildet ist und Ausnehmungen sowie mindestens einen Einfassungssteg für den Filter aufweist.
Eine gute Filterwirkung bei gleichzeitig geringem Strömungswiderstand wird dadurch erreicht, dass der Filter elektrostatisch aufgeladen ist.
Das Beatmungsgerät mit Hygienefiltersystem zeichnet sich durch folgende Eigenschaften und Funktionen aus:
- Schutz des gesamten Geräts gegenüber Bakterien und Viren und, oder Partikeln
- Filterwechsel ist wahlweise mit oder ohne Werkzeug durch den Anwender möglich
- Betrieb mit verschmutztem Filter muss unmöglich sein
- Filterwechselintervall wird vom Gerät , abhängig von der Betriebsdauer, vorgegeben

Folgende Spezifikationen des Hygienefiltersystems können gemäß einer Ausführungsform erreicht werden:
- Abscheidegrad von 99,99 % bei einer Partikelgröße von 0,027 µm
- Filtergröße von ca. 10 cm² bei einem maximalen Volumenstrom von 60 l/min
- Widerstand bei 60 l/min maximal 5 cm H₂O
Für andere Filtergrößen können Abwandlungen realisiert werden.
Der Hygienefilter wird in eine Filterklappe eingesetzt und dort abgedichtet, um zu verhindern, dass ungefilterte Nebenluft in das Beatmungsgerät gelangt.
Die Ermittlung des Verschmutzungsgrades des Hygienefilters erfolgt über eine Widerstandsmessung und/oder über einen Betriebsstundenzähler, der mit Einsetzen eines neuen Filters immer auf Null gesetzt wird. Ein Sensor zur Erfassung des Filterwiderstandes ermittelt den Durchgangsflow. Es erfolgt eine Auswertung des Durchgangsflows über die Zeit um einen abnehmenden Flow zu erkennen. Ab einem definierbaren Grenzwert wird die Filterwechselanzeige aktiviert.
Hydrophobe Filter sind undurchlässig für Flüssigkeiten. Die Verwendung kleiner Faserabstände und die Verwendung flüssigkeitsabweisender Fasern bedingen die hydrophoben Eigenschaften. Dadurch ergeben sich bei diesen Filtern keine Einschränkung der Filterleistung und keine Erhöhung des Widerstands im feuchten Milieu. Hydrophobe Filter halten Partikel ab 20 Nanometer (nm) zurück. Somit werden selbst Viren, welche typisch eine Größe zwischen 20 und 300 nm aufweisen, zurückgehalten. Bakterien und deren Sporen weisen eine typische Größe im Bereich 500 nm bis 2000 nm auf und werden somit von hydrophoben Filtern sicher zurückgehalten.
Bei elektrostatischen Filtern ist das Filtermedium negativ und positiv geladen. Die Porenweite ist größer als bei hydrophoben Filtern, weshalb bei gleicher Durchströmungsrate ein geringerer Widerstand besteht. Im Luftstrom enthaltene Bakterien oder Viren werden aufgrund ihrer Oberflächenladung und unabhängig von deren Größe vom geladenen Filtermaterial angezogen und so zurückgehalten.

Offenbarungsgemäß ist auch die Verwendung einer Kombination aus beiden Filtertypen vorgesehen.
Ein Lösungsansatz ist ein grobporiges Filtermaterial, welches über eine elektrostatische Ladung verfügt, um Bakterien und Viren zu filtern.
Das erfindungsgemäße Hygiene-Filtermaterial kann eine hohe Durchflussrate von bis zu 60 l/min auf einer Fläche von weniger als 20 cm2 bei gleichzeitig nahezu absoluter Filterleistung (99,9%) bezogen auf Viren und Bakterien aufweisen.
Zudem wird der Widerstand des Filters kaum von Feuchtigkeit beeinflusst. Der Widerstand überschreitet einen Wert von 5cm H2O bei einem Flow von 60 l/s nicht.
Für den Geräteeingangsfilter, d.h. eine Filtervorrichtung für den Luftansaugbereich einer Vorrichtung zur Beatmung muss eine sehr gute Abdichtung an den Schnittstellen zum Gerät erreicht werden. Nebenluft, d.h. Volumenstromteile die nicht den Filter passieren, muss unbedingt vermieden werden, um keimarmes Atemgas, d.h. Atemgas mit möglichst wenigen Viren, Bakterien und, oder Feststoffpartikeln sicherzustellen. Zudem müssen Handhabung und Bedienbarkeit des Filtersystems im Wartungsfall so einfach und robust wie möglich gestaltet sein. Klare, unverwechselbare mechanische Schnittstellen sowie günstige Materialwahl sollen die Positionierungsgenauigkeit sicherstellen. Die Positionierungsgenauigkeit ist unter anderem verantwortlich dafür, dass der gesamte Volumenstrom ohne Nebenluft durch den Filter strömt.
Die vorliegende Erfindung ist für Notfallbeatmungsgeräte, Heimbeatmungsgeräten oder Klinikbeatmungsgeräten sowie für Inhalationsgeräte und Sauerstoffgeräte geeignet.

In den Zeichnungen sind Ausführungsbeispiele der Offenbarung schematisch dargestellt. Es zeigen:
- Fig. 1: eine perspektivische Ansicht einer Vorrichtung zur Beatmung mit Hygienefiltersystem,
- Fig. 2: ein Hygienefiltersystem in einem ersten Ausführungsbeispiel in einer Explosionsdarstellung,
- Fig. 3: ein Hygienefiltersystem in einem ersten Ausführungsbeispiel in einer perspektivischen Schnittdarstellung,
- Fig. 4: ein Hygienefiltersystem in einem zweiten Ausführungsbeispiel in einer perspektivischen Explosionsdarstellung,
- Fig. 5: ein Hygienefiltersystem in einem zweiten Ausführungsbeispiel in einer dreidimensionalen Schnittdarstellung,
- Fig. 6: ein Hygienefiltersystem in einem dritten Ausführungsbeispiel in einer perspektivischen Explosionsdarstellung,
- Fig. 7: ein Hygienefiltersystem in einem dritten Ausführungsbeispiel in einer perspektivischen Schnittdarstellung,
- Fig. 8: ein Hygienefiltersystem in einem vierten Ausführungsbeispiel in einer perspektivischen Explosionsdarstellung,
- Fig. 9: ein Hygienefiltersystem in einem vierten Ausführungsbeispiel in einer perspektivischen Schnittdarstellung,
- Fig. 10: ein Hygienefiltersystem in einem fünften Ausführungsbeispiel in einer perspektivischen Darstellung,

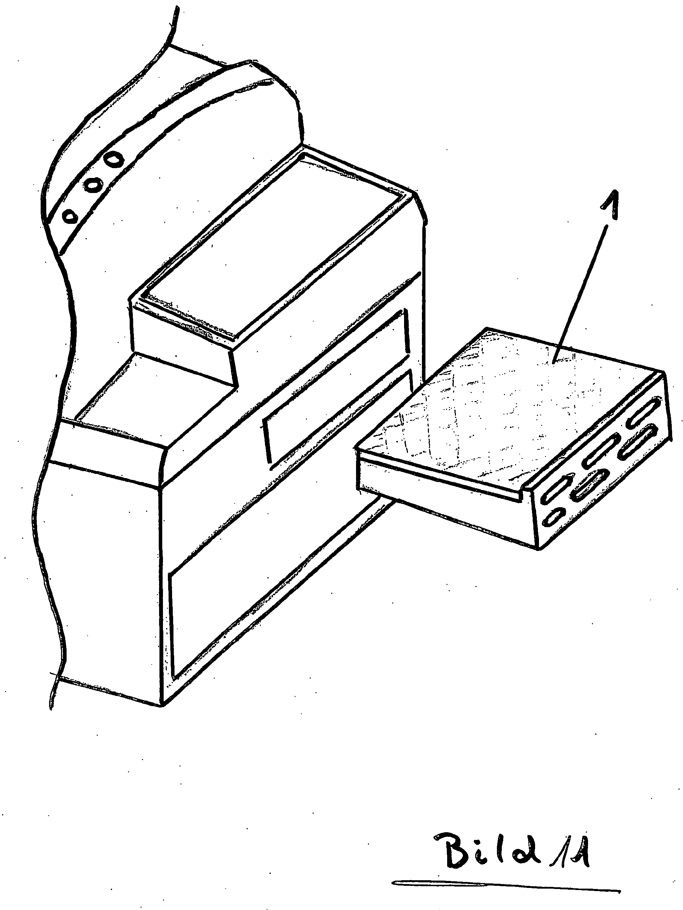

- Fig. 11: eine Ausführungsvariante, bei der durch den Filter eine Dichtung bereitgestellt wird und

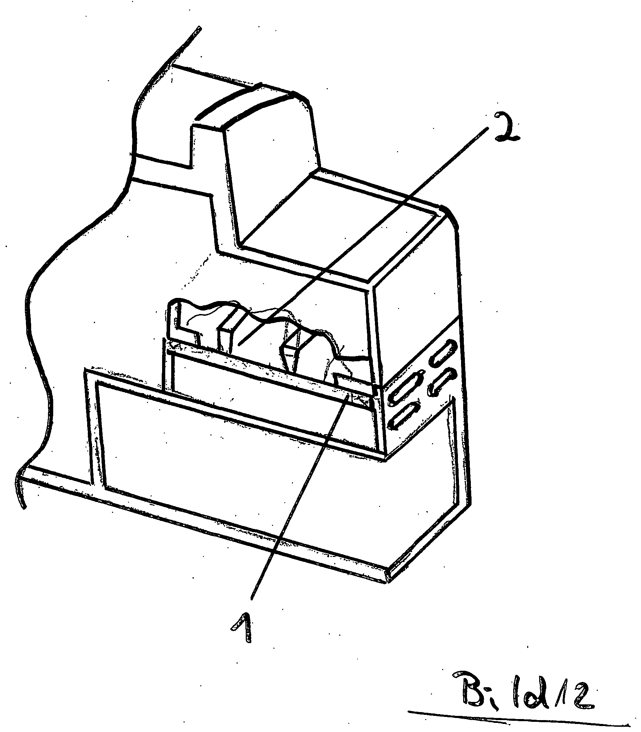

- Fig. 12: eine Ausführungsform ähnlich zu Fig. 11, bei der Leitrippen verwendet werden, die den Filter auf einer vorgebbaren Position halten.

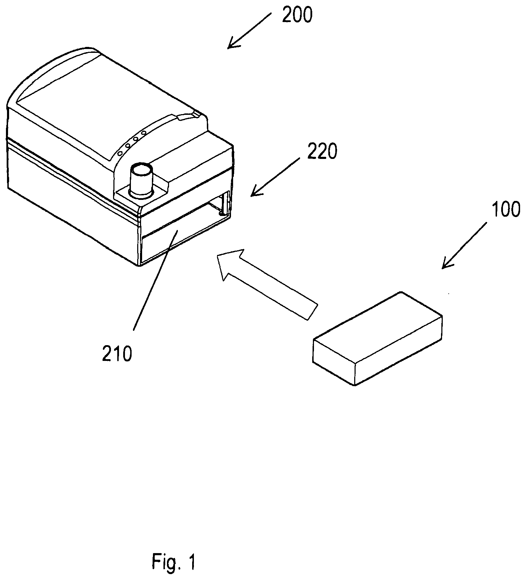

Figur 1 bildet eine perspektivische Ansicht einer Vorrichtung zur Beatmung (200) mit Hygienefiltersystem (100) ab. Das Hygienefiltersystem (100) wird in dem in Figur 1 gezeigten Ausführungsbeispiel in eine Halterungsvorrichtung (210) innerhalb des Luftansaugbereiches (220) einer Vorrichtung zur Beatmung (200) eingesetzt.

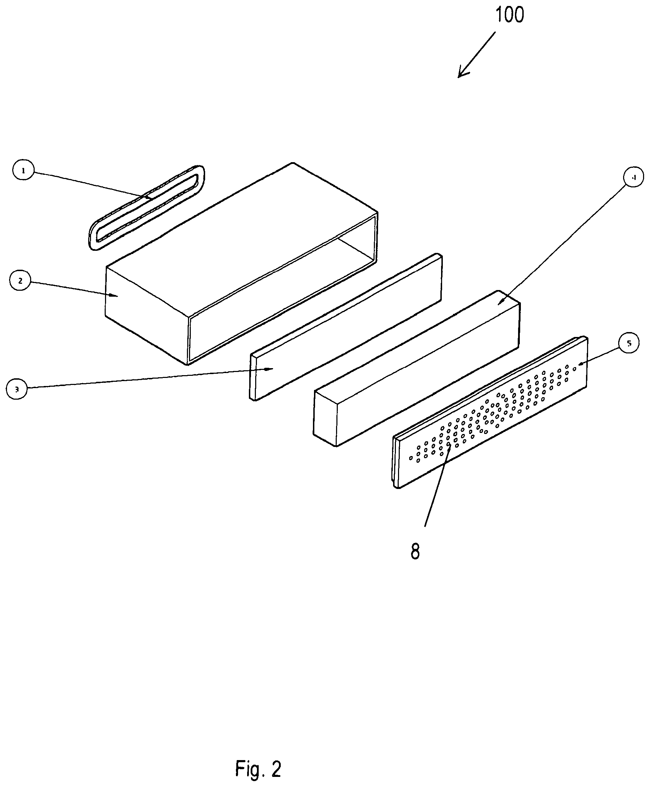

Figur 2 zeigt das Hygienefiltersystem (100) in einem ersten Ausführungsbeispiel in einer perspektivischen Explosionsdarstellung. In diesem ersten Ausführungsbeispiel ist das Hygienefiltersystem (100) innerhalb eines Gehäuses (2) angeordnet und besteht weiterhin im Wesentlichen aus wenigstens einem Feinfilter (3) und wenigstens einem in Volumenstromrichtung vor dem Feinfilter (3) angeordneten Grobfilter (4).
Der Ansaugdeckel (5) schließt das Gehäuse (2) zur Umgebung hin ab und ist derart ausgestaltet, dass erste gröbste Verunreinigungen in der anzusaugenden Umgebungsluft zurückgehalten werden - hier beispielsweise realisiert durch eine Vielzahl von Ansaugbohrungen (8) mit geeignetem Durchmesser kleiner als die zurückzuhaltenden Gröbstverunreinigungen. An dem Gehäuse (2) des Hygienefiltersystems (100) in Einbaurichtung zur Vorrichtung zur Beatmung (200) angeordnet ist eine Gehäuseöffnung (9) (nicht dargestellt in Figur 2) mit einer Dichtung (1) vorgesehen.

Vorzugsweise ist die Gehäuseöffnung (9) gegenüberliegend des Ansaugdeckels (5) positioniert.
Die Gehäuseteile können in Abhängigkeit des gewählten Materials zusammengeklickt, verschweißt oder geklebt sein. Vorzugsweise ist der Ansaugdeckel (5) lösbar mit dem Gehäuse (2) verbunden, um den Filterwechsel oder dessen Reinigung durch die sich dadurch ergebende Zugänglichkeit zu unterstützen.

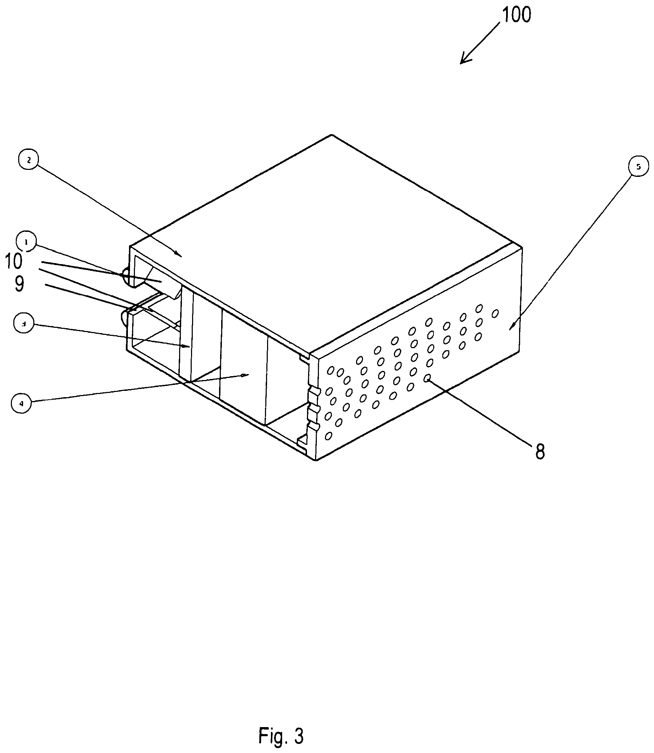

Figur 3 zeigt das erste Ausführungsbeispiel des Hygienefiltersystems (100) in einer dreidimensionalen Schnittdarstellung mit der bevorzugten Anordnung von Grobfilter (4) und Feinfilter (3) in Volumenstromrichtung gesehen hintereinanderliegend. Durch die Ansaugbohrungen (8) des Ansaugdeckels (5) wird Umgebungsluft angesaugt. Um Nebenluft wirksam zu verhindern ist das Gehäuse (2) an seiner durch eine Gehäuseöffnung (9) zur Vorrichtung zur Beatmung (200) hin gebildeten Schnittstelle mit einer Dichtung (1) ausgestattet. Um die Stabilität des Gehäuses (2) insbesondere an der Schnittstelle zur Vorrichtung zur Beatmung (200) hin zu verbessern können, Rippen (10) als Versteifungsstege vorgesehen sein. Dadurch wird eine Dichtflächenverformung reduziert, die Ursache für Undichtigkeiten und damit Nebenluft sein kann. In einer bevorzugten Ausgestaltung der Dichtung (1) ist diese aus Silikon oder auf Silikonbasis hergestellt.

Das erste Ausführungsbeispiel des Hygienefiltersystems (100) ist derart ausgestaltet, dass die Lage der Gehäuseöffnung (9) außermittig stirnseitig zu der Gehäusekontur vorgesehen ist. Durch die außermittige Lage kann das Hygienefiltersystem (100) nur in der einen gewünschten Position und Orientierung in die Halterungsvorrichtung (210) der Vorrichtung zur Beatmung (200) eingesetzt werden - auf diese Weise ist die Handhabung und Bedienbarkeit des Filtersystems im Wartungsfall so einfach und robust wie möglich und bietet eine eindeutige, definierte unverwechselbare mechanische Schnittstelle. Ein lageverkehrter Einbau ist auf diese Weise ausgeschlossen und unterstützt den Betrieb ohne Nebenluft.

Gemäß einer anderen Ausführungsvariante ist der Hygienefilter (100) symmetrisch ausgebildet und kann in beiden Lagen eingebaut werden.

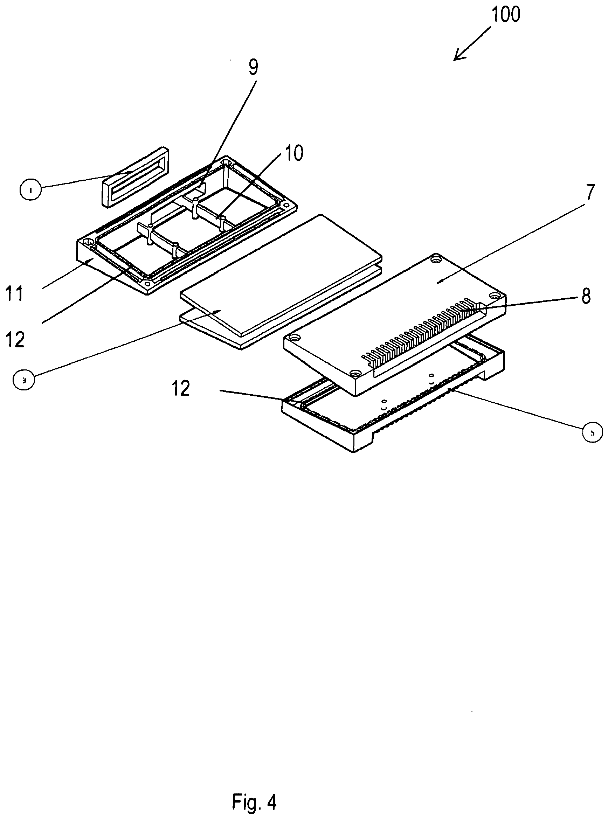

Figur 4 bildet das zweite Ausführungsbeispiel des Hygienefiltersystems (100) in einer perspektivischen Explosionsdarstellung ab. Anders als im ersten Ausführungsbeispiel gemäß der Figuren 2 und 3 ist das Gehäuse in Längsrichtung geteilt und aus wenigsten einem Ansaugdeckel (5), einem Filterdeckel (7) sowie einem Filtermaterialhalter (11) gebildet. Der Filter (3) im zweiten Ausführungsbeispiel ist durch Vliesmaterial gebildet und kann in einer bevorzugten Ausgestaltung elektrostatische Eigenschaften aufweisen. Das elektrostatische Filtervlies (3) ist bahnförmig ausgebildet und innerhalb des Gehäuses (5, 7) keilförmig angeordnet. Die Fixierung des Filters (3) im und relativ zum Gehäuse erfolgt durch Klemmung zwischen dem Filtermaterialhalter (11) und jeweils dem Ansaugdeckel (5) beziehungsweise dem Filterdeckel (7).

Der Filtermaterialhalter (11) kann innenseitig mit wenigstens einem Distanzhalter (10) beispielsweise in Rippenform ausgestattet sein um zum einen die mechanische Stabilität des rahmenförmigen Filtermaterialhalters (11) zu erhöhen und das vorzugsweise elektrostatische Filterflies (3) in der gewünschten Ebene zu halten und ein Einfallen in den Filtermaterialhalter (11) zu verhindern.

Durch die keilförmige Anordnung des Filtervlieses (3) innerhalb des Hygienefiltersystems (100) anstelle der rechtwinkeligen Ausrichtung im Gehäuse ist die nutzbare Filteroberfläche nicht durch den Gehäusequerschnitt an dieser Stelle beschränkt und kann im Rahmen der bekannten mathematischen Zusammenhänge der Trigonometrie größer ausfallen.

Auch das zweite Ausführungsbeispiel des Hygienefiltersystems (100) ist derart ausgestaltet, dass die Lage der Gehäuseöffnung (9) außermittig stirnseitig zu der Gehäusekontur vorgesehen ist, um den Einbau des Hygienefiltersystem (100) nur in der einen gewünschten Position und Orientierung in die Halterungsvorrichtung (210) der Vorrichtung zur Beatmung (200) zu unterstützen.

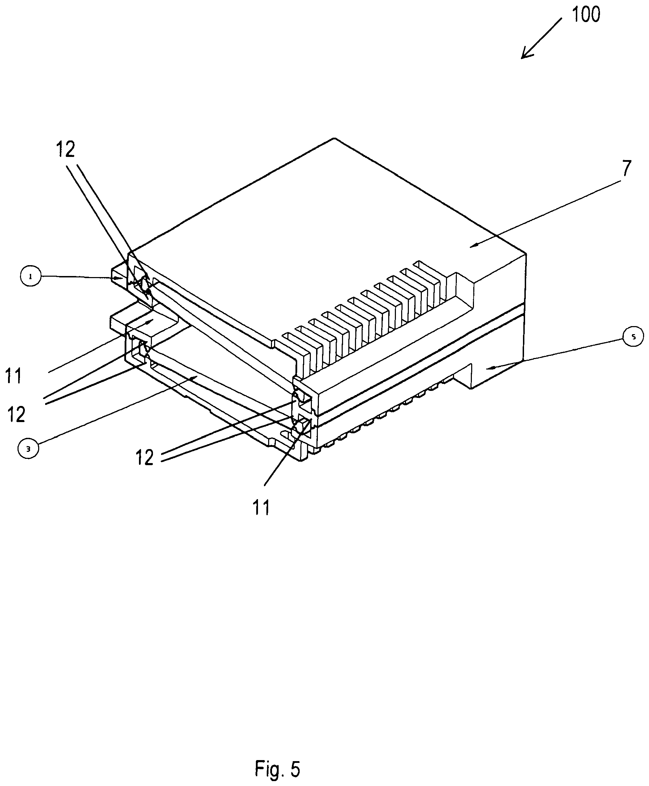

Figur 5 zeigt das zweite Ausführungsbeispiel des Hygienefiltersystems (100) in einer dreidimensionalen Schnittdarstellung mit der keilförmigen Anordnung des Filtervlieses (3) innerhalb des Hygienefiltersystems (100) anstelle der rechtwinkeligen Ausrichtung im Gehäuse und die sich daraus ergebende gegenüber dem Gehäusequerschnitt deutlich, d.h. eine bis zu ca. Faktor vier bis fünf vergrößerte nutzbare Filteroberfläche. Infolgedessen kann die Filterfläche in etwa 10.000 mm² betragen, wenn der lichte Querschnitt des Gehäuses 2000 bis 2500 mm² beträgt.
Die das Gehäuse bildenden Elemente (5, 7, 11) werden bevorzugt durch Zusammenklicken miteinander verbunden. Um eine zuverlässige Klemmung des Filterflieses (3) zu gewährleisten können die gehäusebildenden Elemente (5, 7, 11) additiv oder wahlweise Stege (12) in Form von Gehäusekanten aufweisen. Dadurch ist gewährleistet, dass das Risiko von neben Luft reduziert ist. Die Stege (12) können derart angeordnet sein, dass das Filtervlies (3) einseitig oder beidseitig geklemmt wird.

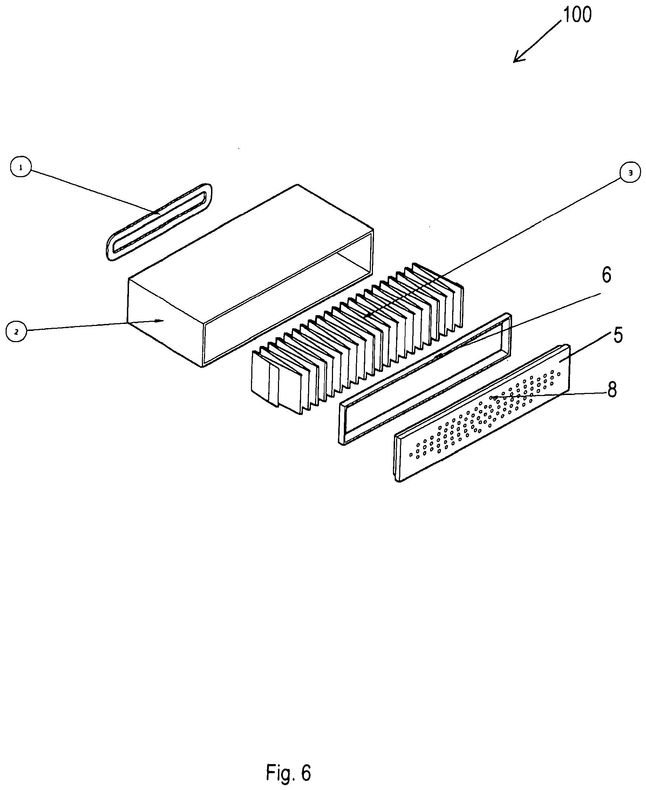

Figur 6 bildet das Hygienefiltersystem (100) in einem dritten erfindungsgemäßen Ausführungsbeispiel in einer perspektivischen Explosionsdarstellung ab. Der Aufbau des Gehäuses (2) ist analog dem ersten Ausführungsbeispiel dargestellt in Figur 2 und 3. Abweichend davon ist das Filtermaterial gefaltet und die Faltlagen sind beabstandet zueinander angeordnet, sodass das Filtermaterial mit seiner Oberfläche auch zwischen den Faltlagen eine für den Filtrierungsprozess aktive Fläche bereitstellt.

Der Faltfilter (3) kann auf diese Weise eine bis zu ca. Faktor sieben vergrößerte nutzbare Filteroberfläche als die Konstruktion gemäß Ausführungsbeispiel zwei und eine bis zu ca. Faktor fünfunddreißig vergrößerte nutzbare Filteroberfläche als die Konstruktion gemäß Ausführungsbeispiel eins bereitstellen. Infolgedessen kann die Filterfläche in etwa 70.000 mm² betragen, wenn der lichte Querschnitt des Gehäuses 2000 bis 2500 mm² beträgt.
Der Faltfilter (3) ist derart innerhalb des Gehäuses orientiert, sodass der Volumenstrom des zu filternden Mediums radial auf die jeweiligen Faltkanten einströmt.
Die Gehäuseteile können in Abhängigkeit des gewählten Materials verschweißt oder geklebt sein. Vorzugsweise ist der Faltfilter (3) durch eine Vergussmasse (6) innerhalb des Gehäuses (2) festgelegt und abgedichtet, sodass Nebenluft weitgehend verhindert wird. Bei dem Hygienefiltersystem (100) gemäß dem dritten Ausführungsbeispiel ist daher ein Filtermaterialwechsel in der Regel nicht vorgesehen.

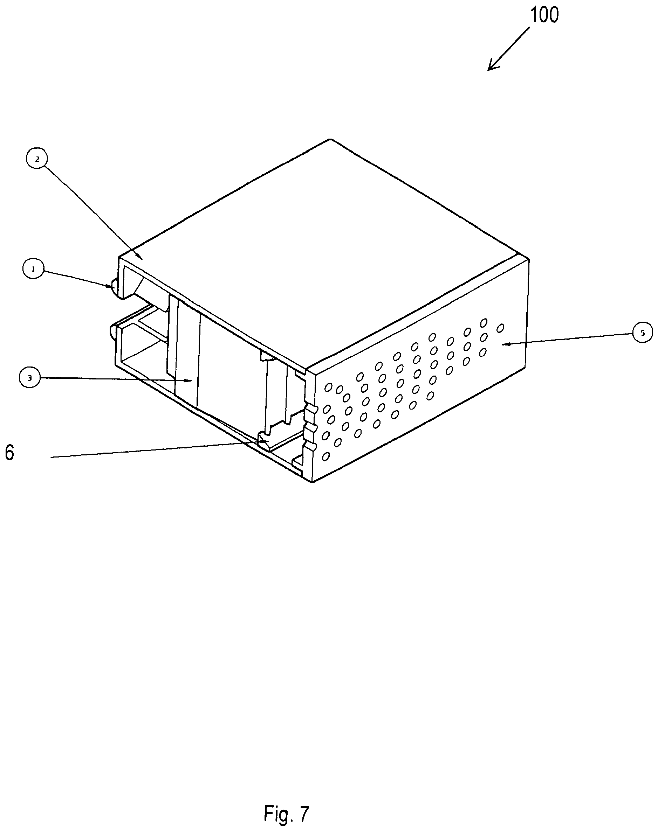

Figur 7 zeigt die Situation gemäß Figur 6 als dreidimensionale Schnittdarstellung.

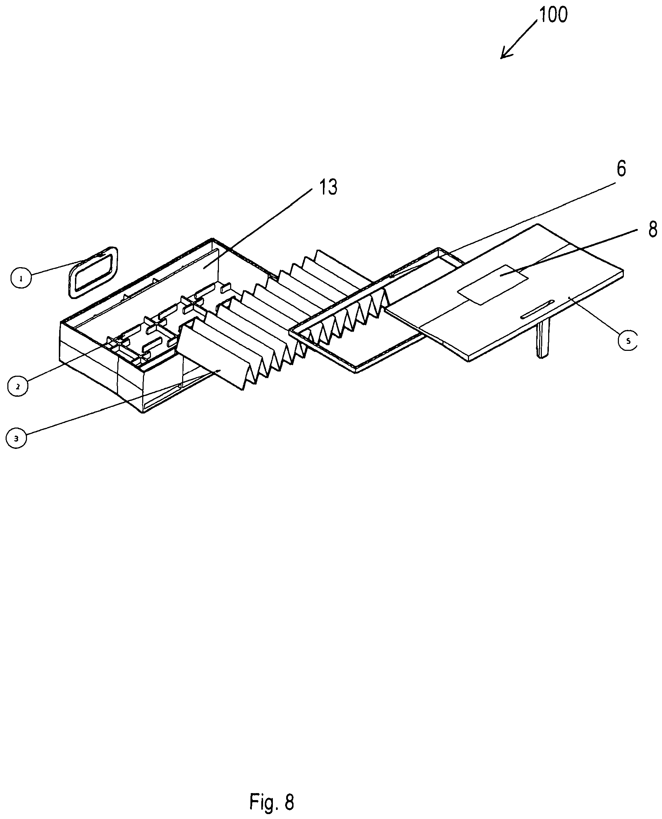

Figur 8 bildet das Hygienefiltersystem (100) in einem vierten Ausführungsbeispiel in einer perspektivischen Explosionsdarstellung ab.
Es wird ein Faltfilter (3) verwendet, der mit seinen Faltkanten parallel zur Längsausrichtung des Hygienefiltersystems (100) innerhalb des Gehäuses (3) und per Vergussmasse (6) gegen unerwünschte Nebenluft abgedichtet festgelegt ist.
Der Faltfilter (3) ist auch im vierten Ausführungsbeispiel derart innerhalb des Gehäuses orientiert, dass der Volumenstrom des zu filternden Mediums radial auf die jeweiligen Faltkanten einströmt.

Um diese radiale Anströmung der Filterkanten durch den Volumenstrom des zu filternden Mediums zu realisieren sind zwei unterschiedliche Möglichkeiten vorgesehen: Ist ein Ansaugdeckel (5) stirnseitig und gegenüberliegend der Gehäuseöffnung (9) verwendet wird der Volumenstrom durch geeignete innenseitige Gehäusewandungen (13) geführt und in geeigneter Weise doppel-s-förmig geleitet.

Ist ein Ansaugdeckel (5) - wie dargestellt - deckseitig und gegenüberliegend der Gehäuseöffnung (9) verwendet strömt das zu filternde Medium über die Ansaugöffnung (8) des Ansaugdeckels 5)in einem Winkel zur Gehäuseöffnung (9) in das Hygienefiltersystem (100) und strömt die Filterkanten radial an. In diesem Fall wird der Volumenstrom durch geeignete innenseitige Gehäusewandungen (13) geführt und in geeigneter Weise s-förmig geleitet.

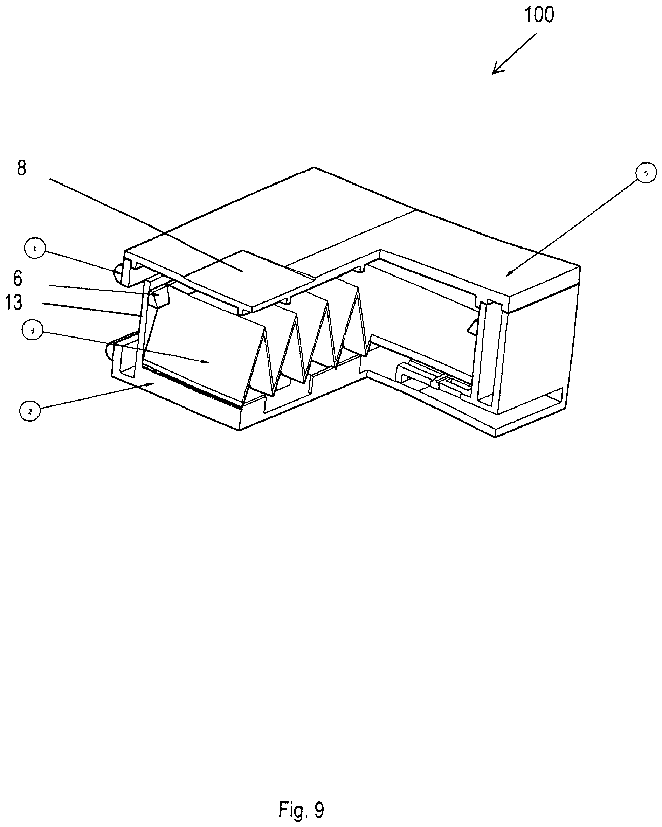

Figur 9 zeigt die Situation gemäß Figur 8 als dreidimensionale Schnittdarstellung.

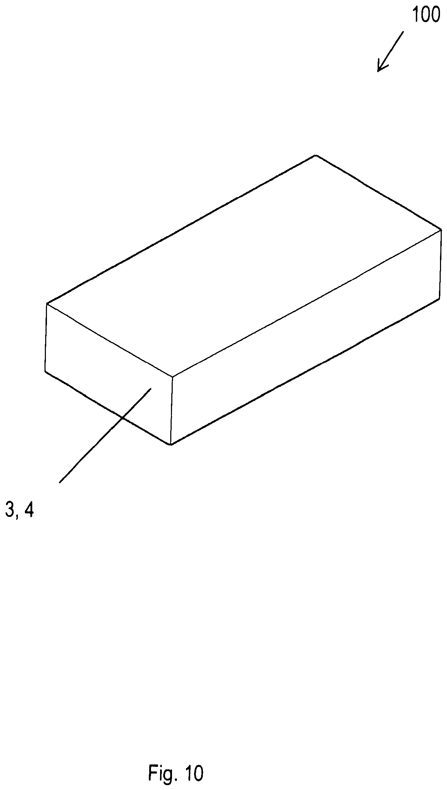

Figur 10 zeigt das Hygienefiltersystem (100) in einem fünften Ausführungsbeispiel in einer perspektivischen Darstellung. Das Hygienefiltersystem (100) ist in diesem Ausführungsbeispiel ohne Gehäuse (3) realisiert und besteht im Wesentlichen aus wenigstens einem geeigneten Filtermaterial das, um als kompatible Alternative zu den vorangegangenen Ausführungsbeispielen in der Halterungsvorrichtung (210) für ein Hygienesystem (100) einer Vorrichtung zur Beatmung (200) einsetzbar zu sein muss der Filter (3, 4) im Fall inelastischer Filtermaterialeigenschaften in seinen Außenabmessungen im Wesentlichen denen der Gehäuseabmessungen der vorangegangen Ausführungsbeispiele entsprechen oder kann im Fall elastischer Filtermaterialeigenschaften in seinen Außenabmessungen größer ausfallen. Das Filtermaterial kann auf einem oder auch mehreren Werkstoffen bestehen.

Für die Problematik der unerwünschten Nebenluft ist es besonders vorteilhaft, erfindungsgemäß der Filter (3, 4) über elastische Filtermaterialeigenschaften verfügt, übermaßig zu den Gehäuseabmessungen ausgebildet ist und das Filtermaterial erfindungsgemäß über Abdichtungseigenschaften verfügt. Durch die resultierende wenigstens teilweise elastische oder elastisch-plastische Stauchung des Filters (3, 4) durch die Verringerung der Abmessungen des Hygienesystems (100) beim Einsetzen in die Halterungsvorrichtung (210) für ein Hygienesystem (100) einer Vorrichtung zur Beatmung (210) legt sich das Filtermaterial in abdichtender Weise an die Halterungsvorrichtung (210) an und reduziert das Nebenluftrisiko.
Je nach Konditionierung eines Werkstoffes oder Anordnung mehrerer Werkstoffe können im Ganzen oder partiell unterschiedliche Filtereigenschaften vorliegen, die in Filterungen unterschiedlicher Partikel oder Mikrolebewesen münden. Beispielsweise kann eine Schaummatratze mit homogenem oder inhomogenem Strukturaufbau verwendet sein, sodass die Filterwirkung im Ganzen oder bereichsweise unterschiedlich ist. Unterschiedliche Werkstoffkonditionierungen können beispielsweise elektrostatische oder, und antibakteriellen Eigenschaften sein.
Bei einer Fertigung der Dichtung zum Gerät aus einem elektrostatischen Filtermaterial kann auch bei einer leichten Undichtigkeit eine ausreichende Keimfreiheit unterstützt werden. Bei einer entsprechenden elektrostatischen Aufladung werden Viren und/oder Bakterien elektrostatisch vom Filtermaterial angezogen. Zusätzliche konstruktive Varianten bestehen in der Bereitstellung einer Keilform und/oder der Verwendung von Luftleitrippen.

Erfindungsgemäß wird bei einer entsprechenden keilförmigen Konstruktion das Filtermaterial durch eine keilförmige Gestaltung des Gehäuses gegen die Dichtfläche angepresst.

## Patentansprüche

1. Hygienefilter (100) für eine Vorrichtung zur Beatmung (200), aufweisend wenigstens einen Filter (3,4) zur Filterung von Mikroorganismen und, oder Feststoffpartikeln aus einem Atemluftvolumenstrom, bei dem das Filtermaterial als Abdichtungsmittel gegenüber den atemluftvolumenstromleitenden Elementen (2, 210) ausgebildet ist, sodass das Risiko auftretender Nebenluft reduziert ist, wobei der Hygienefilter (100) durch ein Gehäuse (2) gebildet wird, das den Filter (3, 4) einhaust und ein atemluftvolumenstromleiatemluftvolumenstromleitendes Element bildet, wobei das Gehäuse (2) derart ausgebildet ist, das es in eine Halterungsvorrichtung (210) der Vorrichtung zur Beatmung (200) einsetzbar ist, **dadurch gekennzeichnet, dass** das Gehäuse (2) eine keilförmige Gestaltung aufweist, und dadurch das Filtermaterial gegen eine Dichtfläche angepresst wird.

2. Hygienefilter (100) nach Anspruch 1, **dadurch gekennzeichnet, dass** der Filter (3, 4) aus einem oder mehreren Werkstoffen aufgebaut ist und über homogene oder partiell unterschiedliche mechanische Eigenschaften und Filterwirkungen verfügt.

3. Hygienefilter (100) nach Anspruch 1, **dadurch gekennzeichnet, dass** das Filtermaterial eine Schaummatratze oder ein elektrostatisches Filtervlies ist.

4. Hygienefilter (100) nach Anspruch 1, **dadurch gekennzeichnet, dass** das Filtermaterial gefaltet ist.

5. Hygienefilter (100) nach Anspruch 1, **dadurch gekennzeichnet, dass** der Filter aus wenigstens einem Grobfilter (4) und wenigstens einem Feinfilter (3) besteht.

6. Hygienefilter (100) nach Anspruch 1, **dadurch gekennzeichnet, dass** der Filter (3, 4) gegenüber dem Gehäuse (2) zusätzlich zur Abdichtung durch das Filtermaterial mit einer Vergussmasse (6) abgedichtet ist.

7. Hygienefilter (100) nach Anspruch 1, **dadurch gekennzeichnet, dass** der Atemluftvolumenstrom durch geeignete innenseitige Gehäusewandungen (13) innerhalb des Gehäuses (2) doppel-s-förmig oder s-förmig leitbar ist.

8. Hygienefilter (100) nach Anspruch 1, **dadurch gekennzeichnet, dass** das Gehäuse (2) wenigstens eine Gehäuseöffnung (9) aufweist, die außermittig stirnseitig zu der Gehäusekontur angeordnet ist, sodass der Hygienefilter (100) nur in einer Position und Orientierung in die Halterungsvorrichtung (210) der Vorrichtung zur Beatmung (200) einsetzbar ist.

9. Hygienefilter (100) nach Anspruch 1, **dadurch gekennzeichnet, dass** das Gehäuse (2) wenigstens eine Rippe (10) aufweist, sodass eine Versteifung des Gehäuses (2) unterstützt ist.

10. Hygienefilter (100) nach Anspruch 9 **dadurch gekennzeichnet, dass** die wenigstens eine Rippe (10) zusätzlich als Distanzhalter ausgebildet ist.

11. Vorrichtung zur Beatmung (200) aufweisend eine Halterungsvorrichtung (210) innerhalb des Luftansaugbereiches (220), **dadurch gekennzeichnet, dass** der Hygienefilter (100) nach einem der Ansprüche 1 bis 11 in die Halterungsvorrichtung einsetzbar und verbindbar ist, sodass das Risiko auftretender Nebenluft reduziert ist.

12. Vorrichtung zur Beatmung (200) nach Anspruch 11, **dadurch gekennzeichnet, dass** der Hygienefilter (100) über mindestens einen Teil der Halterungsvorrichtung abgedichtet ist.

13. Vorrichtung nach Anspruch 12, **dadurch gekennzeichnet, dass** die Dichtung aus einem elektrostatischen Filtermaterial ausgebildet ist.

## Claims

1. Hygiene filter (100) for a ventilation apparatus (200), having at least one filter (3, 4) for filtering microorganisms and/or solid particles from a breathing air volume flow, wherein the filter material is configured as a sealing means with respect to the elements (2, 210) that guide the breathing air volume flow, such that the risk of secondary air occurring is reduced, the hygiene filter (100) is formed by a housing (2) which accommodates the filter (3, 4) and forms an element that guides the breathing air volume flow, wherein the housing (2) is configured so as to be insertable into a holding apparatus (210) of the ventilation apparatus (200), **characterized in that** the housing (2) has a wedge-shaped design, and as a result the filter material is pressed against a sealing surface.

2. Hygiene filter (100) according to Claim 1, **characterized in that** the filter (3, 4) is constructed from one or more materials and has homogeneous or partially different mechanical properties and filter effects.

3. Hygiene filter (100) according to Claim 1, **characterized in that** the filter material is a foam mattress or an electrostatic filter fleece.

4. Hygiene filter (100) according to Claim 1, **characterized in that** the filter material is folded.

5. Hygiene filter (100) according to Claim 1, **characterized in that** the filter consists of at least one coarse filter (4) and at least one fine filter (3).

6. Hygiene filter (100) according to Claim 1, **characterized in that**, in addition to the sealing by the filter material, the filter (3, 4) is sealed off from the housing (2) by a casting compound (6).

7. Hygiene filter (100) according to Claim 1, **characterized in that** the breathing air volume flow is able to be guided in a double S-shaped or S-shaped manner within the housing (2) by suitable internal housing walls (13).

8. Hygiene filter (100) according to Claim 1, **characterized in that** the housing (2) has at least one housing opening (9) which is arranged off-center on the end side of the housing contour, such that the hygiene filter (100) is insertable into the holding apparatus (210) of the ventilation apparatus (200) in only one position and orientation.

9. Hygiene filter (100) according to Claim 1, **characterized in that** the housing (2) has at least one rib (10) such that stiffening of the housing (2) is supported.

10. Hygiene filter (100) according to Claim 9, **characterized in that** the at least one rib (10) is additionally configured as a spacer.

11. Ventilation apparatus (200) having a holding apparatus (210) within the air intake region (220), **characterized in that** the hygiene filter (100) according to one of Claims 1 to 11 is insertable into the holding apparatus and connectable, such that the risk of secondary air occurring is reduced.

12. Ventilation apparatus (200) according to Claim 11, **characterized in that** the hygiene filter (100) is sealed off via at least a part of the holding apparatus.

13. Apparatus according to Claim 12, **characterized in that** the seal is formed from an electrostatic filter material.

## Revendications

1. Filtre hygiénique (100) pour un dispositif respiratoire (200), présentant au moins un filtre (3, 4) pour le filtrage de microorganismes et/ou de particules solides hors d'un courant volumique d'air respiratoire, dans lequel le matériau de filtre est constitué par un moyen d'étanchéité à l'égard des éléments (2, 210) conduisant le courant volumique d'air respiratoire, de telle manière que le risque d'admission d'air secondaire soit réduit, dans lequel le filtre hygiénique (100) est formé par un boîtier (2), qui héberge le filtre (3, 4) et forme un élément conduisant le flux volumique d'air respiratoire, dans lequel le boîtier (2) est réalisé de telle manière qu'il puisse être inséré dans un dispositif de support (210) du dispositif respiratoire (200), **caractérisé en ce que** le boîtier (2) présente une configuration en forme de coin, et que de ce fait le matériau de filtre est pressé contre une face d'étanchéité.

2. Filtre hygiénique (100) selon la revendication 1, **caractérisé en ce que** le filtre (3, 4) est construit en un ou plusieurs matériau(x) et présente des propriétés mécaniques et des performances de filtrage homogènes ou partiellement différentes.

3. Filtre hygiénique (100) selon la revendication 1, **caractérisé en ce que** le matériau de filtre est un matelas de mousse ou une nappe de filtre électrostatique.

4. Filtre hygiénique (100) selon la revendication 1, **caractérisé en ce que** le matériau de filtre est plié.

5. Filtre hygiénique (100) selon la revendication 1, **caractérisé en ce que** le filtre se compose d'au moins un filtre grossier (4) et d'au moins un filtre fin (3).

6. Filtre hygiénique (100) selon la revendication 1, **caractérisé en ce que** le filtre (3, 4) est rendu étanche par rapport au boîtier (2) par une masse de scellement (6) en plus de l'étanchéité par le matériau de filtre.

7. Filtre hygiénique (100) selon la revendication 1, **caractérisé en ce que** le courant volumique d'air respiratoire peut être conduit en forme de double S ou en forme de S à l'intérieur du boîtier (2) par des parois de boîtier intérieures appropriées (13).

8. Filtre hygiénique (100) selon la revendication 1, **caractérisé en ce que** le boîtier (2) présente au moins une ouverture de boîtier (9), qui est disposée frontalement en position décentrée par rapport au contour du boîtier, de telle manière que le filtre hygiénique (100) ne puisse être inséré que dans une position et une orientation dans le dispositif de support (210) du dispositif respiratoire (200).

9. Filtre hygiénique (100) selon la revendication 1, **caractérisé en ce que** le boîtier (2) présente au moins une nervure (10), de telle manière que la rigidité du boîtier (2) soit soutenue.

10. Filtre hygiénique (100) selon la revendication 9, **caractérisé en ce que** ladite au moins une nervure (10) forme en outre un élément d'écartement.

11. Dispositif respiratoire (200) présentant un dispositif de support (210) à l'intérieur de la zone d'aspiration d'air (220), **caractérisé en ce que** le filtre hygiénique (100) selon l'une quelconque des revendications 1 à 11 peut être inséré et assemblé dans le dispositif de support, de telle manière que le risque d'admission d'air secondaire soit réduit.

12. Dispositif respiratoire (200) selon la revendication 11, **caractérisé en ce que** le filtre hygiénique (100) est rendu étanche au moyen d'au moins une partie du dispositif de support.

13. Dispositif selon la revendication 12, **caractérisé en ce que** le joint d'étanchéité est formé en un matériau de filtre électrostatique.
